# EUROPEAN PATENT SPECIFICATION

(11) **EP 0 991 812 B1**
(45) Date of publication and mention of the grant of the patent: **12.05.2004**
(21) Application number: 98912873.1
(22) Date of filing: 30.03.1998
(51) Int. Cl.: D21H 17/66, A61L 15/18, D21C 9/00, D21H 21/14

(54) **METHOD FOR THE PREPARATION OF CELLULOSE PULP, CELLULOSE PULP TO BE USED IN ABSORPTION PRODUCTS, AND ABSORPTION PRODUCT**
VERFAHREN ZUR ZELLSTOFFHERSTELLUNG, ZELLSTOFF FÜR SAUGFÄHIGE PRODUKTE UND SAUGFÄHIGE PRODUKTE
PROCEDE DE PREPARATION DE PATE A PAPIER, PATE A PAPIER DESTINEE A ETRE UTILISEE DANS DES PRODUITS ABSORBANTS ET PRODUIT ABSORBANT

(30) Priority: 07.05.1997 SE 9701711
(43) Date of publication of application: 12.04.2000
(73) Proprietor: Stora Enso Aktiebolag, 791 80 Falun (SE)
(72) Inventor: NORLANDER, Leif, S-791 61 Falun (SE)
(74) Representative: Johansson, Lars E.
(86) International application number: PCT/SE1998/000585
(87) International publication number: WO 1998/050629

(56) References cited:
- WO-A-91/05106
- WO-A-91/05108
- FILE WPI, Derwent Accession No. 87-149264, LENGD FORESTRY ACAD., "Prepn. of Paper from Pulp - Involves Using Potash Alum and/or Aluminium Sulphate to Increase Absorbency while Maintaining Wet Strength"; & SU,A,1 261 996 (07-10-86) DW8721.

## Description

### TECHNICAL FIELD

The invention relates to a method for the preparation of a cellulose pulp adapted to be defibrated (fluffed) for production of absorbent materials intended to be included as a constituent in absorbent products. The invention also relates to a method for the preparation of a defibrated cellulose pulp suitable for use in production of absorbent materials intended to be included as a constituent in absorbent products. The invention also relates to the prepared cellulose pulp which is suitable for defibration as well as the defibrated and thereafter compressed pulp. Also the absorbent products which are produced by use of the cellulose pulp are included in the invention.

### BACKGROUND OF THE INVENTION

In order to increase the comfort properties of typical absorbent products as diapers, sanitary napkins, incontinence protection and other hygenic articles, and also in order to decrease the volume in connection with storage and transport, absorbent products are made thinner and thinner. Furthermore, there is a continuous aim to increase the rate of production. These conditions put increasingly higher demands on the ability to defibre and compress of the fluff pulp. These demands have however been hard to combine, since the result of defibration measured as knot content and network strength is impaired at increased production per time unit in hammer mills or other defibration equipment. Moreover, increased rates in the production line leads to higher linear loads being required in order to press the fluffed absorption body or the web of absorption material to the desired final density. Another factor to be considered in connection with process and product development in this field is that an increased use of so called super absorbents in absorbent products leads to that the demands on the absorption capacity are decreased. The demands on the strength properties are, on the contrary, increased when the amount of fluff pulp per absorbent product is reduced. Moreover, the fluff pulp is required to have good wicking properties.

Yet an essential factor to be considered is the high consumption of energy in connection with defibration and that the supply of electric energy is limited in large parts of the world and/or that the energy cost is high.

The above factors show a need of a fluff pulp which
- is easy to compact after defibration in a nip in a press or a calender,
- has a high network strength, which enables use of low grammages and thus a saving in the amount of used fluff pulp, and
- can be defibrated with a relatively low input of energy.

### PRIOR ART

In US 5 308 896 a method is described for production of a fibre with high bulk, which is stated to be easy to compress and with particles that attach to the fibre. The fibres have the ability to form hydrogen bonds. Furthermore, the preferred compounds which are added to the pulp have functional groups with ability to form hydrogen bonds with fibres and particles.

EP 0 705 365 describes a method to produce a fluff pulp cross linked in the dry state, where two, three or multiple functional alcohols increase the ability of compression in the cross linked fibre.

In US 5 300 192 and US 5 352 480 methods are described to produce a pulp with the ability to attach particles to fibres which are not cross linked. These fibres can be compressed at pressures lower than 200 pli (8000 psi), (appr 34.9 kN/m, 558 bar). The compounds which are stated to have the ability to bind particles to the fibres have the same ability of hydrogen bonding as the compounds which are intended to be used in the above mentioned US 5 308 896. The compounds are characterised in that they have a volatility which is less than that of water. Polyethylene glycol, polypropylene glycol, polyacrylic acid, polyamides, polyamines, poly(caprolacton)diol and combinations thereof are mentioned as examples.

In EP 0 493 516 tests are presented that show that the wicking properties of CTMP fluff pulp can be improved by impregnating the fibres in water suspension with aluminium salt in aqueous solution at a pH which preferably should be within the range 8.5-9. The improved wicking and rate of absorption is, according to EP 0 577 590, achieved by the attachment of a porous layer on the surface of the fibre. In EP 0 493 516 and EP 0 577 590 the use of similar compounds is recommended in order to achieve the desired effects, aluminium ions in the shape of polyaluminium chloride or sulphate, aluminium phosphate or sodium aluminate or mixtures of these compounds.

### SHORT DESCRIPTION OF THE INVENTION

An increased content of moisture usually leads to that a web, which has been formed of cellulose fibres defibrated in the dry state, becomes easier to compress to a high density. An increased content of moisture is however at the same time impairing the properties of the fluff pulp in connection with defibration. Moreover, it is not possible to add moisture to the defibrated material in fast operating machines for treatment of defibrated cellulose pulp, due to the risk of deposits in tubes, etc. Also, the limited times at hand between formation and press makes it hard to moisturise the formed web or the absorption body in a practical way.

When the content of water (moisture) is increased in a conventional fluff pulp there also arise serious negative effects. These effects may for example present themselves as an increased knot content, increased demand of defibration energy and decreased network strength. The moisture content may however, with only limited negative effects, be increased in order to make it easier to compress the pulp to high density, at the same time as only limited negative effects arise, if the pulp is treated with at least some non-polymeric aluminium salt which is water soluble.

The treatment with said not polymeric aluminium salt should be performed within the pH range of 4-8, preferably at a pH between 5 and 7, most preferably between pH 5.5 and 6.5.

The distinguishing features of the invention are thus, according to one aspect of the invention which relates to the processes and measurements taken in the cellulose mill, to add at least some non polymeric aluminium salt which is water soluble to a stock of cellulose fibres in water suspension, to form this fibre pulp to a web which is dewatered and dried, and to adjust its content of moisture to greater than 8% but not more than 20% at the latest before defibration. The pulp should preferably contain 9-15% of water before the defibration, i.e. when the defibration treatment is started. The purpose of adjusting the moisture content to said relatively high level before defibration is to reassure a suitable content of moisture after the defibration but before the compression. The moisture content should, according to a second aspect of the invention which relates to the handling in connection with the compression of the defibrated (fluffed) pulp, be greater than 4.5% but not more than 15%, preferably greater than 5% and at most 12% after defibration but before compression. It should be noted in connection herewith that a dessication of the fibre material takes place before the web or the absorption body passes the press equipment, in connection with forming processes where the fibre is dispersed in air and the absorption body is formed on a running wire or in pockets. In connection with pressing or calandration, the above stated moisture conditions should be upheld, i.e. that the defibrated pulp shall comprise more than 4.5%, preferably more than 5% moisture and max. 15%, preferably max. 12% moisture.

An additional positive effect with the relatively high content of moisture in the defibrated material in the produced absorption product, is that the product becomes more soft than a corresponding product with a lower content of moisture but with the same density.

The adjustment of the moisture content may be performed in different ways and at different points in the integrated process of cellulose preparation, wet-lap forming and defibration. Normally, the fluff pulp is delivered undefibrated to the customer, who defibrates the pulp and compresses it in connection with the production of the absorbent products in question. It may hereby be conceived that the moisture content of the pulp is adjusted to the desired level before the delivery from the pulp mill, and that the moisture content is maintained at this level during transport and storage up to and including defibration and compression. It may also be conceived that the pulp mill delivers the pulp in a dry state, i.e. with a moisture content below 8%, and that the adjustment of the moisture content to the suitable level is performed instead with the customer before defibration, so that the moisture content after defibration, when a considerable amount of moisture escapes, becomes the above mentioned in the subsequent compression. Also combinations of these two alternatives are conceivable. In practice, a combination of a certain moisturising to above normal content of moisture before delivery from the pulp mill, followed by a complementary addition of water with the customer before defibration and compression, can be made. This alternative has the advantage that e.g. risk of formation of mildew during long time transportation and/or storage of the material with a high content of moisture can be eliminated. As to the performance of the adjustment of moisture content in practice, there are also several different possibilities. The moisture may for example be present as water which has not been dried away in connection with the production of the fluff pulp and/or that moisture is added by spraying or some other technique of application at the latest before defibration. In principle, it may also be conceived to moisturise the defibrated pulp before compression, but this is hard to do in practice and also costly, if the moisturising e.g. should be done by means of superheated steam, which in that case would be the method closest at hand.

Additional characterising features and aspects of the invention are apparent from the appending claims and from the following description of performed tests.

### SHORT DESCRIPTION OF DRAWINGS

In the following description of performed tests, reference is made to the attached drawings, of which
- Fig. 1: in the form of a diagram shows the effect of moisture content on density after compression of different types of pulps,
- Fig. 2: illustrates the density and thereby the compressability as a function of the content of moisture for some different pulps,
- Fig. 3: is a diagram showing the strength of compressed test bodies as wet network strength as a function of moisture content at defibration,
- Fig. 4: shows the dry network strength as a function of the moisture content at defibration,
- Fig. 5: is a diagram showing the knot content as a function of moisture content at defibration,
- Fig. 6: illustrates the absorption capacity of compressed absorption bodies as a function of density, and
- Fig. 7: is a diagram from Paper Chemistry, An Introduction, Dan Eklund, Tom Lindström, DT Paper Science Publications, p.134-144.

### PERFORMED TESTS

Bleached chemical fluff pulp fibres, produced according to the sulphate pulp method, were treated in introductory tests. The fibre raw material was Nordic softwood.

In a first test series, trims from the couch were used cut by the trim squirt, which trims had never been dried, in order to form sheets in the form of sheets according to standard method. The pulp concentration was 0.7%. Addition of aluminium sulphate, Al₂(SO₄)₃ and different salts was made at approximately 1.5% pulp concentration before final dilution in sheet form. The aluminium sulphate chemical was aluminium sulphate as a trade merchandise, which has a dry content of 75%, if nothing else is stated. The aluminium sulphate which was used in the tests was manufactured by Kemira AB. Occurring pH adjustments were made with H₂SO₄ and NaOH, respectively.

The wicking tests were made on an inclined plane with 30° inclination according to a method which has been developed and standardised by the applicant. In short, the method implies that the sample is placed on the inclined plane and that the lower edge of the sample is brought into contact with the sample liquid. The amount of liquid that is absorbed is registered per time unit by weighing during a time period of 900 s, whereafter the sample is cut and strips are cut out across the direction of flow and weighed. Knot content was determined according to SCAN-CM 37:85. Network strength was performed with a metering device of the brand Alwetron with a rate of 60 mm/min on test bodies, 1 g, produced according to SCAN 33 :80. Other fluff properties were analysed with standard methods which are described in WO93/16228 by the same applicant. The defibration was made in a Kamas hammer mill in laboratory scale. The reported defibration energy relates to net energy. The results of the introductory tests are shown in Table 1.

**Table 1**

| | | | | | |
|---|---|---|---|---|---|
| Aluminium sulphate, amount charged | g/kg | 0 | 50 | 75 | 100 |
| pH | | 6.5 | 6.5 | 6.5 | 6.5 |
| Ash content | % | 0.20 | 1.1 | 1.6 | 2.1 |
| Grammage | g/m² | 816 | 834 | 834 | 826 |
| Thickness | mm | 1.50 | 1.49 | 1.50 | 1.46 |
| Density | kg/m³ | 543 | 559 | 556 | 554 |
| Defibration energy 3500 rpm | kJlkg | 156 | 151 | 122 | 111 |
| Network strength | N/1g | 6.8 | 6.8 | 7.5 | 7.7 |
| Knot content | % | 11.6 | 7.4 | 3.7 | 1.8 |
| Wicking on inclined plane, 30° | | | | | |
| inclination | | | | | |
| Density sample body | kg/m³ | 155 | 152 | 150 | 154 |
| Absorption capacity | | | | | |
| 0-3 cm | g/g | 9.5 | 10.4 | 9.8 | 9.3 |
| 3-6 cm | g/g | 8.0 | 8.6 | 8.2 | 8.0 |
| 6-9 cm | g/g | 8.6 | 9.0 | 8.6 | 8.4 |
| 9-12 cm | g/g | 8.9 | 9.0 | 8.9 | 8.4 |
| 12-15 cm | g/g | 9.0 | 8.8 | 9.0 | 8.6 |
| 15-18 cm | g/g | 8.3 | 8.6 | 8.8 | 8.0 |
| 18-21 cm | g/g | 7.8 | 8.1 | 8.1 | 6.6 |
| 21-24 cm | g/g | 6.5 | 5.6 | 6.5 | 6.0 |

From Table 1 it is clear that aluminium sulphate affects the defibration result in a positive way. At high charges of aluminium sulphate, 75-100 kg/ton, as trade merchandise with a dry content of 75% a considerable decrease in knot content and defibration energy is accomplished, but already at a charge of aluminium sulphate in an amount of 50 kg/ton positive results were noted on the defibration. The results indicate that aluminium salt should be added in an amount corresponding to 3-24, preferably 6-12 g Al/kg pulp. The enhanced fibre exposure also lead to an enhanced network strength with a maintained absorption capacity when measuring wicking on an inclined plane.

The tests which are presented in Table 1 were made on sheets of fluff pulp which had been formed at pH 6-7. In order to clarify the role of the pH and in order for the aluminium sulphate addition to give enhanced defibration properties, tests were also made with lower and higher pH-values. Aluminium sulphate was in all cases charged in an amount corresponding to 100-114 kg/ton. The measured result values are presented in Table 2.

**Table 2 -**

| **Influence of pH on the effect of aluminium sulphate addition** | | | | | |
|---|---|---|---|---|---|
| | | 1 | 2 | 3 | 4 |
| Aluminium sulphate | kg/ton | 114 | 114 | 114 | 114 |
| pH in the stock | | 4.1 | 6.0 | 8.0 | 10.0 |
| Grammage | g/m² | 825 | 854 | 861 | 849 |
| Thickness | mm | 1.54 | 1.57 | 1.56 | 1.63 |
| Density | kg/m³ | 536 | 544 | 552 | 521 |
| | | | | | |
| Ash content | . % | 0.32 | 2.4 | 2.4 | 1.0 |
| Defibration energy | kJ/kg | 145 | 101 | 144 | 159 |
| Absorption SCAN | | | | | |
| Specific volume | dm³/kg | 21.2 | 20.4 | 21.0 | 21.2 |
| Specific volume wet | dm³/kg | 7.8 | 7.1 | 7.6 | 7.8 |
| Absorption time | s | 2.6 | 2.1 | 2.1 | 2.1 |
| Absorption capacity | g/g | 9.2 | 8.4 | 9. | 9.2 |
| Knot content | % | 8.6 | 3.2 | 7.1 | 12.0 |

From Table 2 it is clear that the retention of aluminium on the fibres measured as ash content and especially the defibration energy and the knot content are strongly pH related.

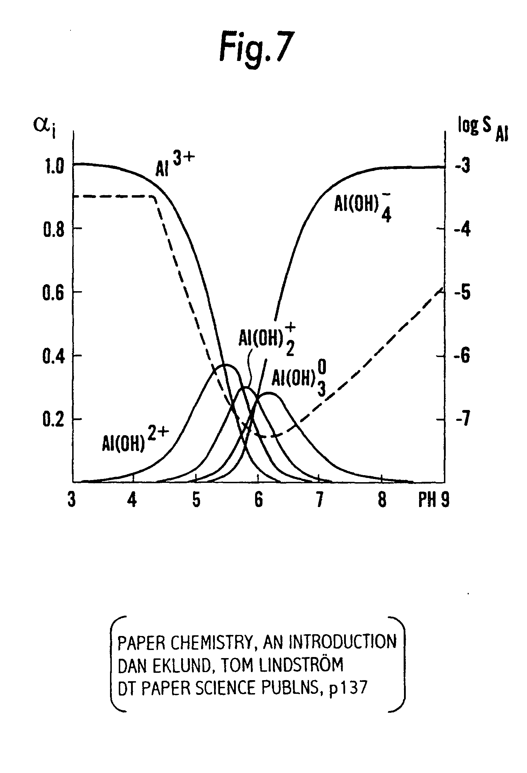

From Table 2 it is also clear that no correspondence to the enhanced defibration properties in sheets of fluff pulp which have been formed at pH 6-7 have been noticed at lower and higher pH values. In Paper Chemistry, An Introduction. Dan Eklund. Tom Lindström. DT Paper Science Publications, P. 134-144 investigations are presented which show that aluminium, from water soluble aluminium salts, is present as different hydroxy complexes at 4<pH<8, preferably pH 5-7, suitably pH 5.5-6.5, see Fig. 7. The presence of these complexes in connection with the forming of the sheet seems to be a prerequisite for enhanced defibration results, which means that also other, non-polymeric aluminium salts, except aluminium sulphate, for example aluminium nitrate, aluminium hydroxide, aluminium oxy hydroxide, aluminium oxy chloride, or other salts of non polymeric aluminium compounds which can be dissolved in an acid or a base in order to form the above mentioned active aluminium complexes at subsequent pH adjustment to the pH interval of 4<pH<8, preferably pH 5-7, suitably pH 5.5-6.5, can be expected to give the desired enhancement in defiberability. This is also confirmed by the relation of aluminium/sulphur in the sheets being considerably higher than in aluminium sulphate, which indicates that said aluminium hydroxy complexes rather than the sulphate ions are the effective part in the dissolved salt. Without binding the invention to a specific theory, the effect on defibration may possibly be explained in that aluminium hydroxy complexes bind to charged groups in cellulose, hemicellulose and lignin residuals, whereby the ability of the fibre to bind to other fibres is affected.

Based on the positive results which have been achieved in connection with the treatment of the pulp with aluminium salt at the adequate pH level, continued tests were made with fluff pulp, based on Nordic softwood fibres of pine and spruce, which were cooked according to conventional sulphate cellulose method and bleached with conventional ECF technique. The pulp was pumped from the bleaching department to a mixing vat at a dry content of appr 1.5%. Al₂(SO₄)₃, appr 9 kg Al/ton, was charged at the inlet to the mixing vat, and was pH adjusted to 6 +/-0.5 with NaOH, appr 38 kg/ton. The total retention time in the mixing vat and in subsequent machine vat(s) was 30-40 min. A sheet was formed on a flat wire and was dewatered in three press nips and was thereafter dried to a moisture content of appr 5%. The machine speed was 65 m/min. The material was cut in a conventional roller machine to a width of 250 mm. The fluff pulp is hereafter denoted LKC (Low Knot Content). Before the test period a reference sample without charge of aluminium salts was taken. This material is hereafter denoted EC. A commercial material, Stora Cell EF, which was produced in the same equipment and with corresponding raw wood material, was also evaluated. This pulp contained appr 0.15 kg/ton of an organic debonder from Akzo Nobel, Berocell 509, and is hereafter denoted EFD. The properties of the materials are described in Table 3 below.

**Table 3**

| | | | |
|---|---|---|---|
| Type of pulp | EC | EFD | LKC |
| Treatment | Untreated | Debonder added | With Al salt |
| Grammage, g/m² | 806 | 807 | 821 |
| Thickness, mm | 1.43 | 1.51 | 1.45 |
| Density, kg/m³ | 562 | 537 | 568 |
| Defibration energy, kJ/kg | 163 | 90 | 107 |
| Network strength, N/1g | 7.3 | 7.5 | 8.4 |
| Aluminium, g/kg pulp | - | - | 8.7 |

Fluff pulps according to Table 3 were moisturised with water spray in a roller machine to varying contents of moisture between appr 5 and 20%. The rolls were wrapped in plastic and were stored for 3 days. After this, the tests were made in pilot equipment for production of a web of fluff pulp. The pilot equipment and its operational data is described below:

The defibration equipment was a hammer mill Nuova Red HM-560 without a sieve, maximum web width = 250 mm equipped with 45 kW motor with stepless controllable rotation speed. The motor was fed via a static frequency changer ABB/Strömberg, Sami 200F380, equipped with equipment for power measuring. The rotation speed was kept constant at 2960 rpm during all tests. The idle power of the mill was 7.5 kW. Air flow through the mill was kept constant at 1200 m³/h, measured with a pitot tube. The feeding in rate of fluff sheet to the hammer mill was kept constant, 25 m/min, which corresponds to a production of 300 kg pulp/h with a moisture content of 6%.

The power on the drive motor for the mill was measured at every test and was calculated and reported as total energy/pulp production with 6% dry content. This way of reporting the consumption of electricity was used independent of the actual moisture content of the fluff pulp.

Absorption bodies were formed on a metal wire and were after formation brought over to a 18 g/m² tissue from Finess AB, B1801. This was used to carry the web through the press. The formation equipment had milling cutter rollers in order to smoothen out unevennesses in formation. The air flow below the wire was kept constant at appr 10600 m³/h and the web speed was kept constant at 34 m/min. The purpose was to reach a final grammage of appr 550 g/m².

The formed web passed between two smooth rollers with a diameter of 226 mm. The pressure between the rollers was adjusted steplessly with pressurised air. Linear load was calculated from the pressure force of the pressurised air cylinders and from the weight of the uppermost roller, which linear load is reported as kN/m.

The air temperature was appr 18-21 °C with a humidity of 20-25% RH at the tests.

### EVALUATION OF THE INFLUENCE OF MOISTURE CONTENT

The material which was produced according to the above and which was studied at different moisture contents, was mainly evaluated with standardised methods. This means that evaluation of fluff properties, except for the determination of moisture content, was made after having conditioned the material during at least 16 hours in a standardised climate, 50% relative humidity, 23°C.

Samples for determination of moisture content were taken and placed as quickly as possible in sealed plastic bags in order to await determination of moisture content. The weight of the samples was determined before and after drying over a night in a hot cabinet at 105°C.

The grammage was determined by cutting samples of 250x250 mm and weighing them. The thickness of the web was determined with a spring loaded micrometer and was determined as mean value from 10 measure points with 20 mm diameter. Density was calculated from grammage and thickness.

Evaluation of punched wet network strength was made in the same way and mainly with the same equipment as was used for measurement of dry network strength. The following deviations in the procedure can be noted. Webs from the pilot machine were conditioned for at least 16 hours in a standardised climate. Circular sample bodies with a diameter of 50 mm were punched out. The sample bodies were weighed and the weight was noted, +/- 1 mg, (the weight of the sample bodies varied between 0.95 and 1.25 g). 4 ml of water was charged to the singular sample bodies and network strength was determined after 2 minutes in a wet state in the same apparatus as the determination of dry network strength. The result was linearly interpolated to 1 g weight for each singular sample body. The mean value of wet network strength was calculated from 10. part samples.

Absorption capacity under a load of 2.5 kPa was determined on sample bodies which had been punched out with 50 mm diameter. The equipment which is used has functions corresponding to SCAN-C 33:80.

Knot content was determined with a Hosokawa Alpine 200 LS-N air jet sieve complemented with a half sphere of Plexiglas, which was used as a lid to encapsulate samples above the sieve cloth. 5 g of fluff pulp were weighed and the sample was separated at 4000 kPa vacuum for 10 minutes. The remainder on 1400 µm (DIN ISO 3310-1) sieve cloth was weighed on a weighing machine. The mean value was calculated from two part samples.

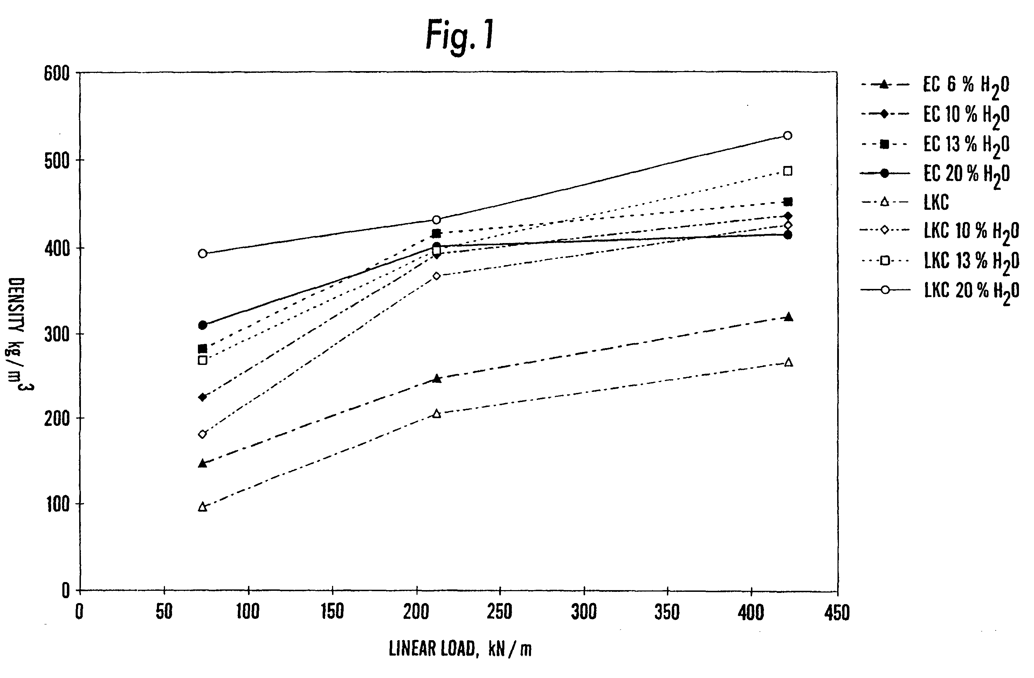

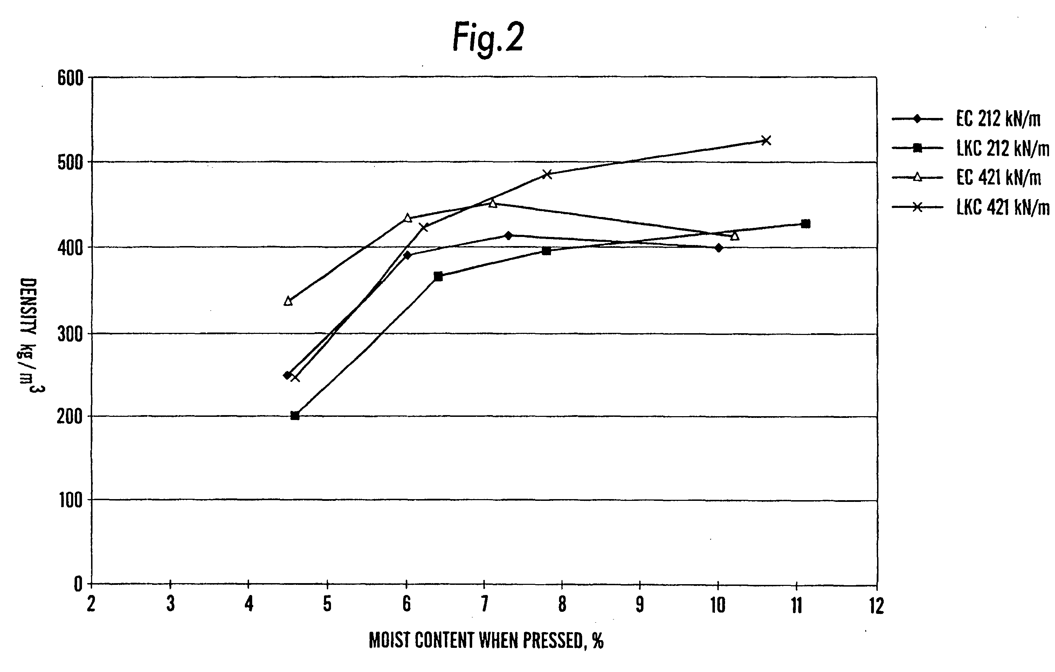

From the Tables 4-6 of the end of the description part and the diagrams in Fig. 1 and 2 it is clear that the compressability is enhanced with the included content of moisture in the fluff pulp, despite the fact that the formation process leads to drying. The sample LKC, which has been treated with a non polymeric aluminium compound, shows already at 10% moisture a compressability which is considerably higher than the corresponding reference samples that have been defibrated at a "normal" content of moisture, appr 6%.

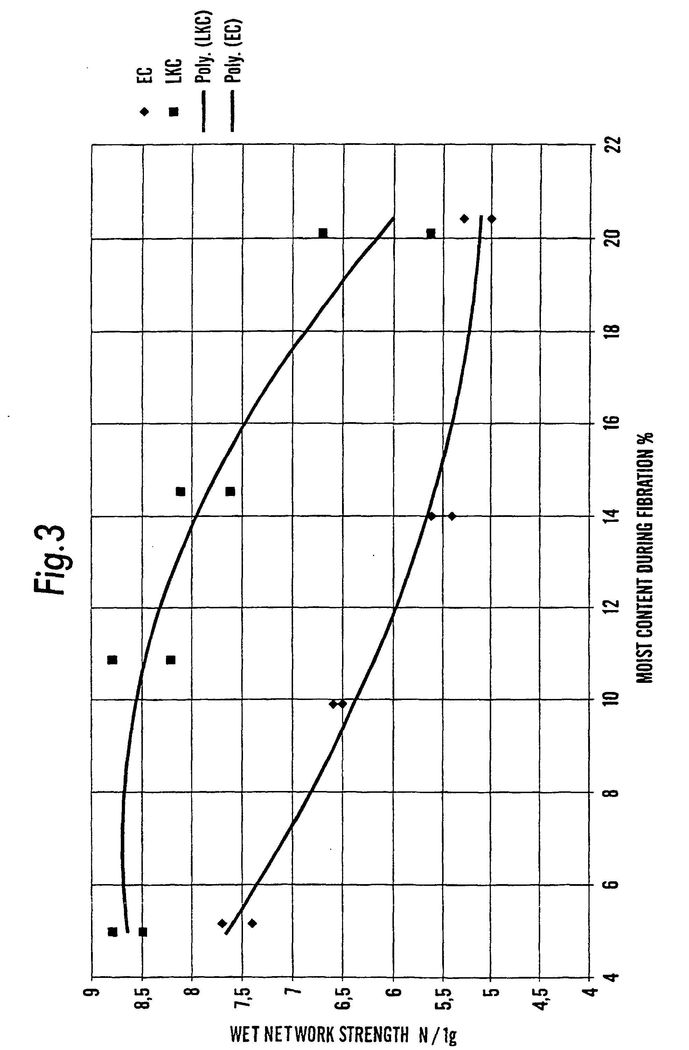

An increased content of moisture in non-defibrated sheets of fluff pulp normally leads to the wet network strength in absorption bodies being strongly impaired. From the diagram in Fig. 3 it is however clear that the impairment is considerably less for samples of fluff pulp of the type LKC, i.e. the sample which has been treated with aluminium salt, than for the reference sample, EC, and that the wet network strength all over is on a higher level in the investigated range of moisture content. Even at such a high moisture content as 20% at defibration, there is a resulting wet network strength in the web of LKC, which corresponds to a strength which is accomplished in connection with defibration at "normal" moisture content for the reference material, EC, appr 6%.

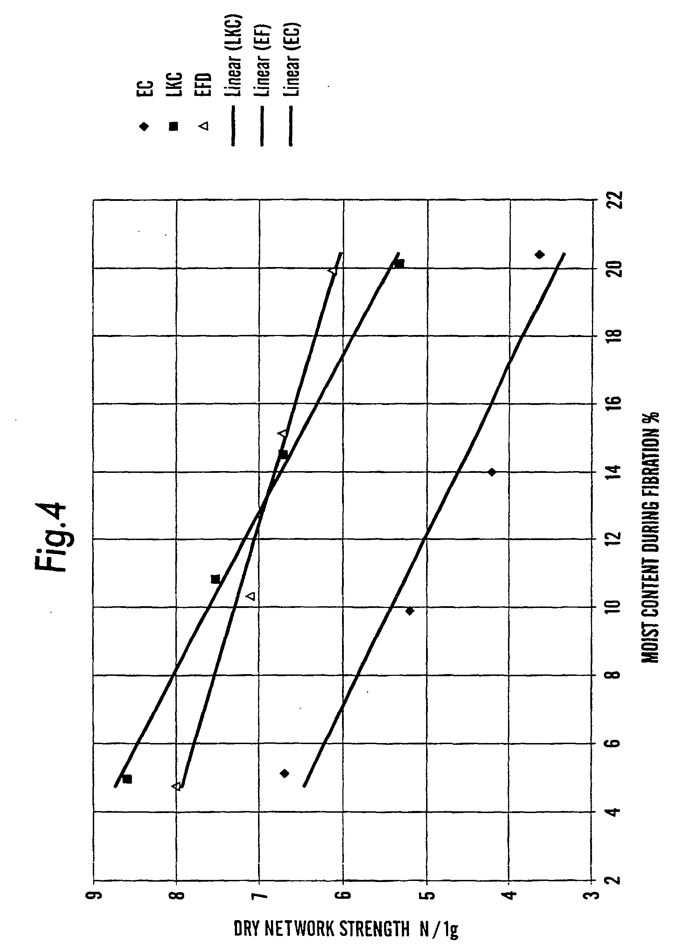

Defibrated, but not pressed fluff pulp was taken out of the formation hood after each test for determination of knot content and network strength. From the diagram in Fig. 4 it is realised that the network strength decreases linearly with increased moisture content for both LKC and EC, and that the difference is constant. Fluff which was defibrated at high moisture contents was however more compacted than the dryer materials. This means that the network strength which is measured in sample bodies which have been formed by hand does not directly reflect the potential of the fluff pulp. Wet network strength measured directly on the web, Fig. 1, can be considered to better reflect the potential of the fluff pulp to give good strength in absorbent products.

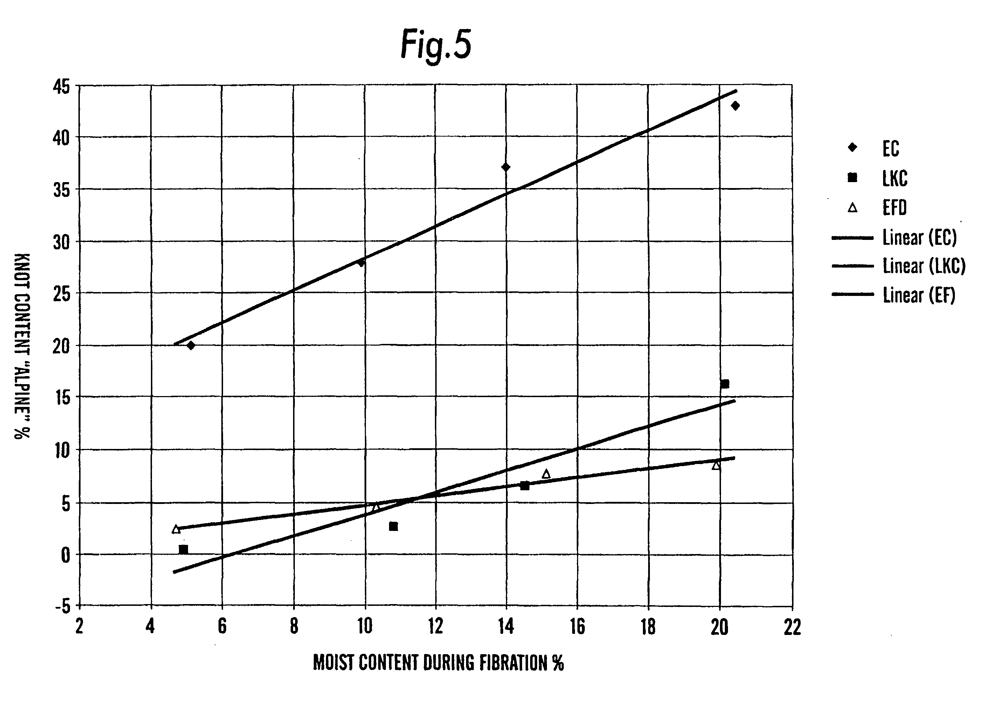

The knot content, which is a measurement of the amount of non-freed fibres, also shows that LKC is more easily defibrated with a good result than are reference samples at comparable moisture contents, see Fig. 5.

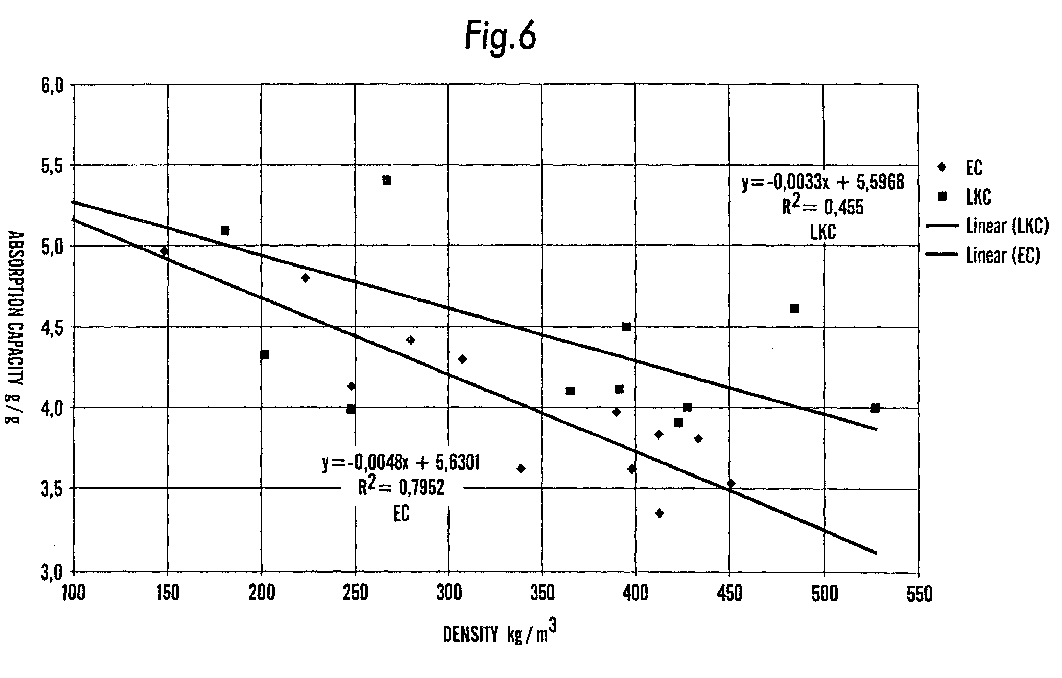

From Fig. 6 it is clear that LKC which is compressed to a high density shows an absorption capacity which corresponds to or is higher than the one which results from the reference EC at a corresponding density.

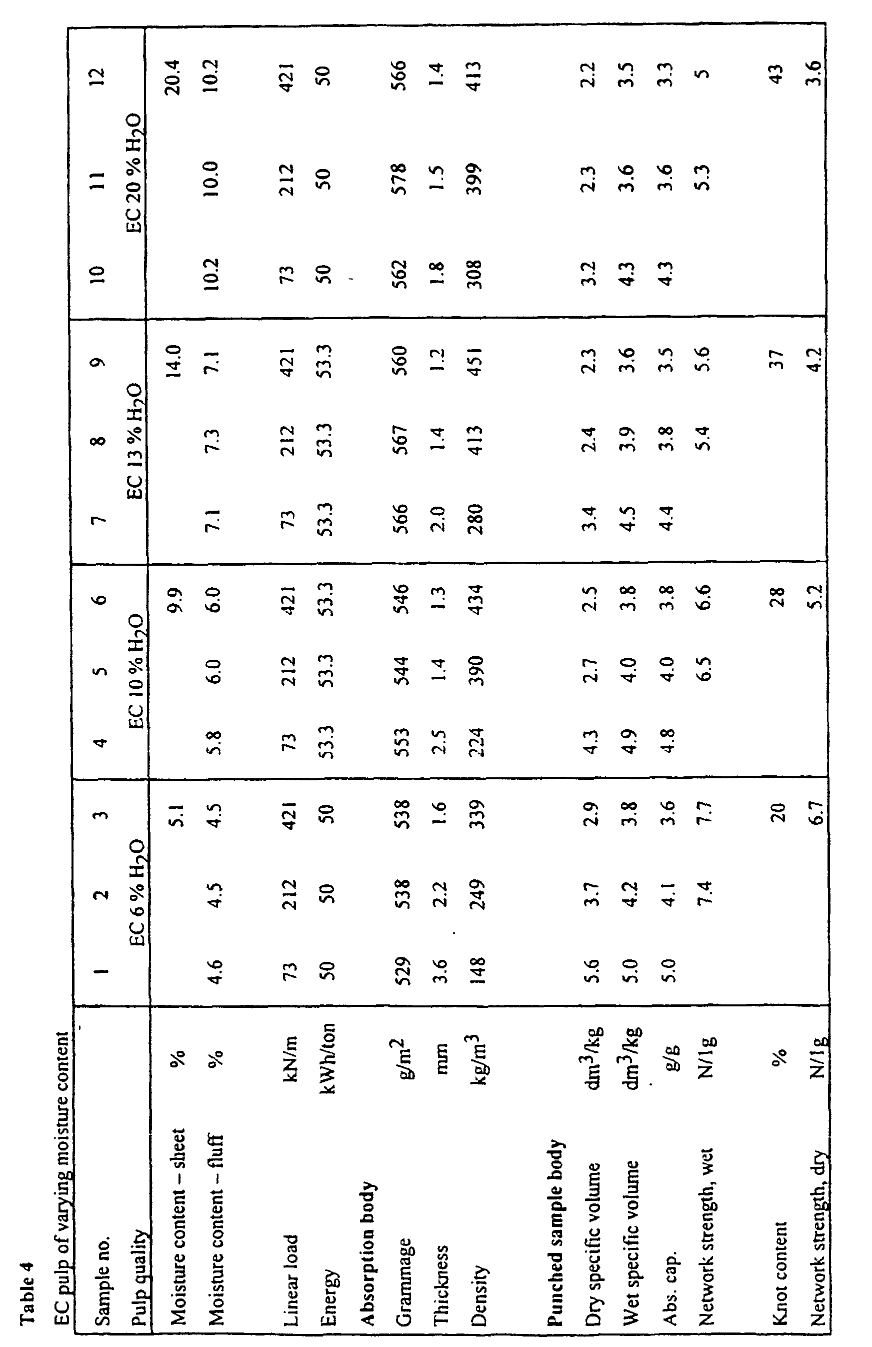

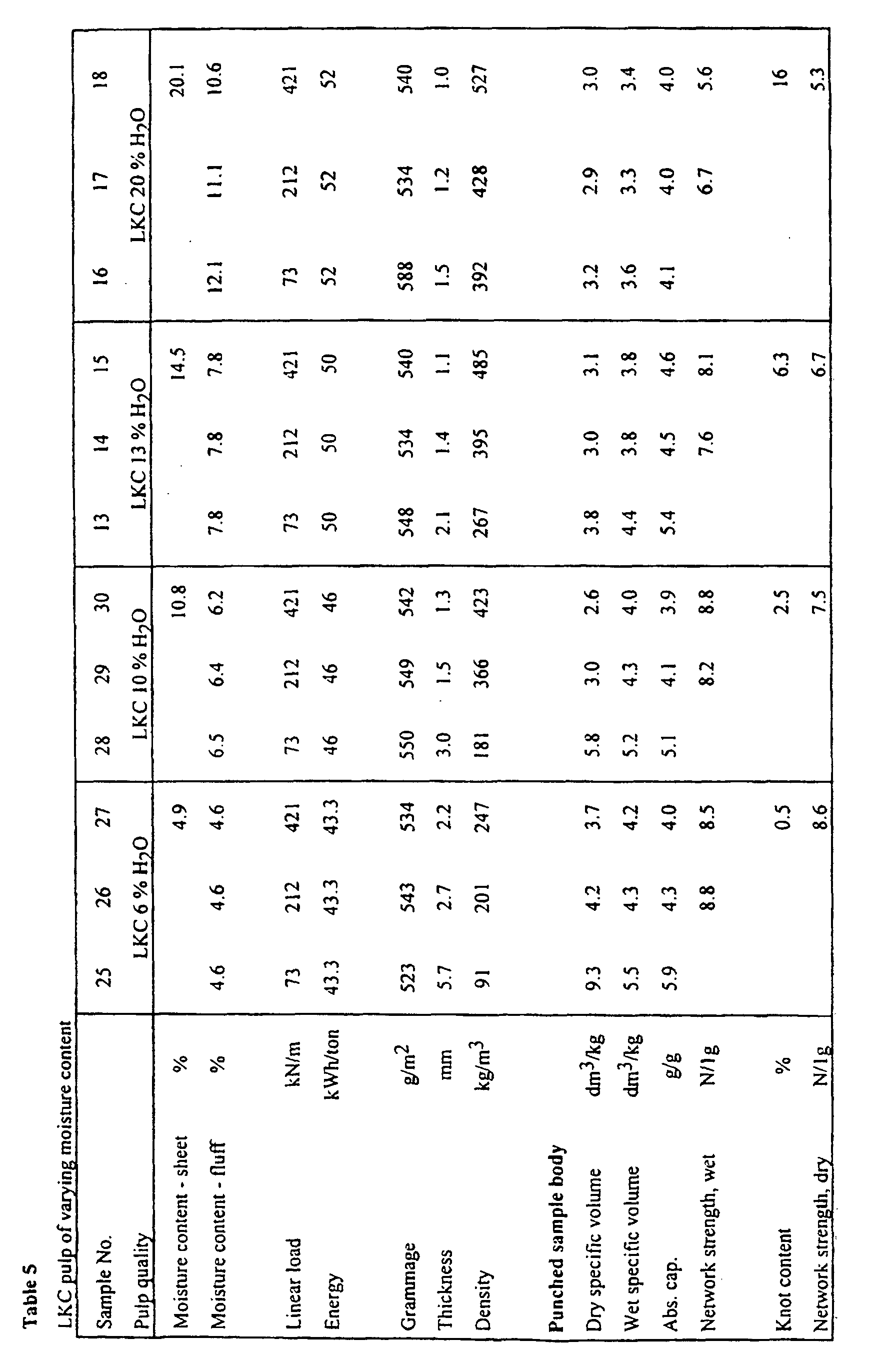

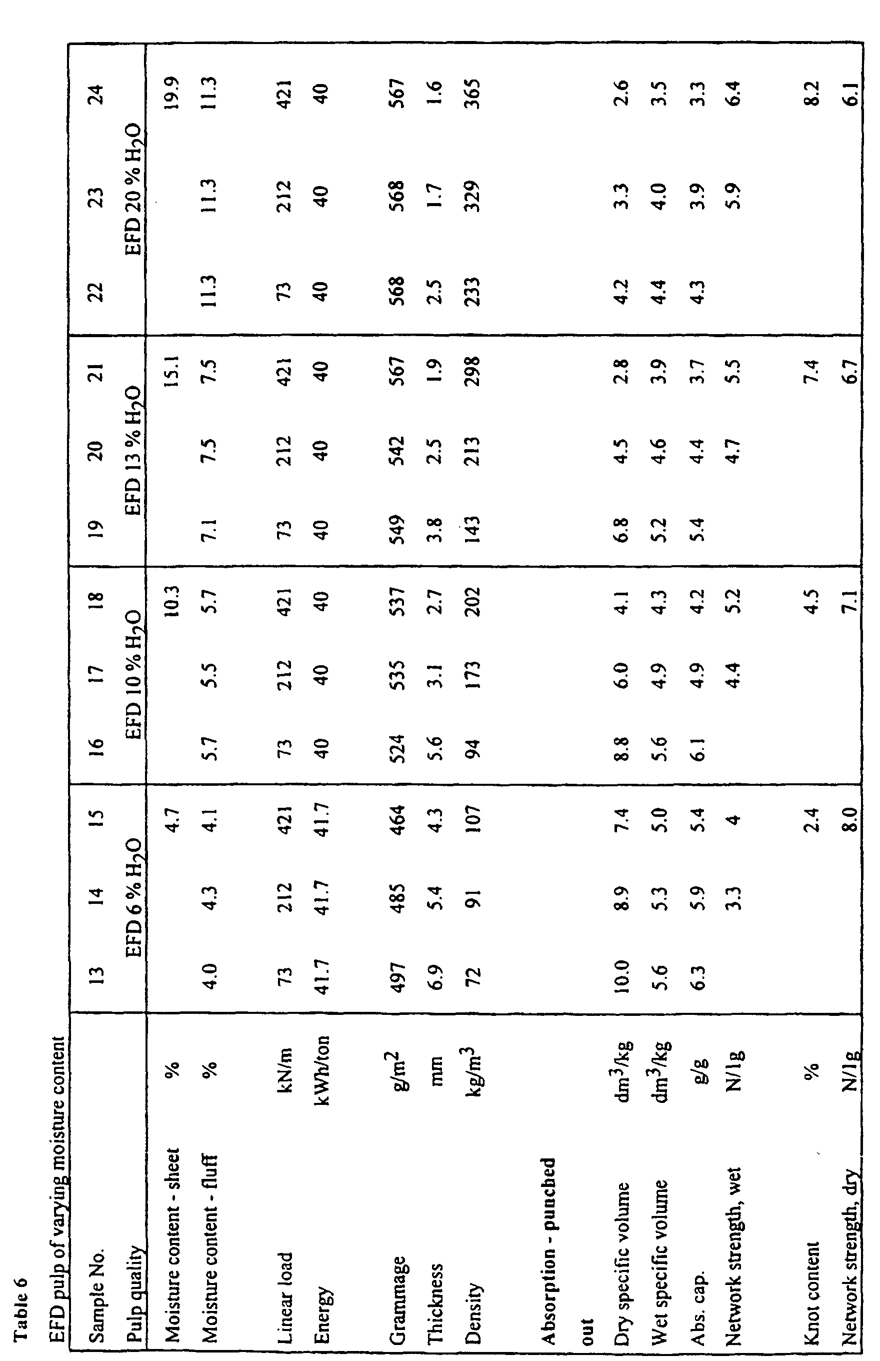

## Claims

1. Method for the preparation of a cellulose pulp adapted to be defibrated (fluffed) for production of an absorbent material intended to be included as a constituent in absorbent products, **characterised in that** at least some non polymeric aluminium salt, which is water soluble, is added to a stock of cellulose fibres in a water suspension, that this fibre pulp is formed to a web which is dewatered and dried, and that its moisture content is adjusted to more than 8% but not more than 20% at the latest before defibration.

2. Method according to claim 1, **characterised in that** the moisture content is adjusted to between 9 and 15%, preferably to at least 10% at the latest before defibration.

3. Method for the preparation of a defibrated cellulose pulp adapted to be used for the production of absorbent material intended to be included as a constituent in absorbent products, **characterised in that** at least some non polymeric aluminium salt, which is water soluble, is added to a stock of cellulose fibres in a water suspension, that this fibre pulp is formed to a web which is dewatered, dried, defibrated and compressed, and that its moisture content is adjusted to more than 4.5% but not more than 15% at the compression which follows after the defibration.

4. Method according to claim 3, **characterised in that** the moisture content is adjusted to be more than 5% but not more than 12% at the compression which follows after the defibration.

5. Method according to any of claims 1-4, **characterised in that** the stock is pH adjusted to 4<pH<8, preferably to pH 6+/-1, suitably to a pH between 5:5 and 6.5.

6. Method according to claim 5, **characterised in that** said aluminium salt is at least some non polymeric aluminium salt, which is water soluble and which in a water solution at said pH forms at least some hydroxy complex with aluminium of the type Al(OH)ₙ^{x}, where n is a number between 1 and 3 and x is 0, + or 2+, that said salt in water solution is brought to act on the cellulose fibres in said suspension at said pH during a time period of at least 2 minutes, and that the fibre pulp is thereafter formed to a web which is dewatered and dried.

7. Method according to claim 6, **characterised in that** said hydroxy complex is one or more of the complexes which comprise Al⁽OH)₃⁰, Al(OH)₂⁺ and Al(OH)²⁺.

8. Method according to any of claims 5-7, **characterised in that** said salt in water solution is brought to act on the cellulose fibres at said pH during a time period of 5-60 min.

9. Method according to any of claims 1-8, **characterised in that** said salt is at least one of the salts aluminium sulphate, aluminium nitrate, aluminium hydroxide, aluminium oxy hydroxide, aluminium oxy chloride or other salts of non polymeric aluminium compounds which can be dissolved in an acid or a base in order to form said active aluminium hydroxy complexes in connection with the subsequent pH adjustment to the range 4<pH<8.

10. Method according to claim 9, **characterised in that** the salt is aluminium sulphate.

11. Method according to any of the preceding claims, **characterised in that** said aluminium salt is added to the stock in an amount corresponding to 3-24 g Al/kg pulp (fibres), preferably in an amount corresponding to 6-12 g Al/kg pulp.

12. Method according to any of claims 1-11, **characterised in that** the cellulose pulp is a chemical cellulose pulp.

13. Method according to claim 12, **characterised in that** the cellulose pulp is a bleached (delignified) cellulose pulp which has been produced according to the sulphate cellulose method.

14. Method according to any of claims 12-13, **characterised in that** the cellulose pulp is a blend of chemical pulp and CTMP.

15. Method according to any of claims 12-14, **characterised in that** the cellulose pulp is blended with synthetic fibres , including the type of synthetic fibres which belong to the group rayon-, polyester-, polypropylene-, polyethene- or so called bond fibres which can be activated by heat.

16. Cellulose pulp adapted to be defibrated (fluffed) for production of an absorbent material intended to be included as a main constituent in absorbent products, characterised in that it has been treated in a water suspension at 4<pH<8 with a non polymeric aluminium salt, which is water soluble, during a time period of at least 2 min, whereafter the fibre pulp has been formed to a web which has been dewatered, dried and had its moisture content adjusted to more than 8% but not more than 20% moisture content.

17. Cellulose pulp according to claim 16, **characterised in that** it has a moisture content of at least 10%.

18. Cellulose pulp according to claim 16 or 17, **characterised in that** the fibres in the thus treated cellulose pulp can be defibrated by defibration at said moisture content with an input of defibration energy not exceeding 130 kJ/kg dry matter, preferably not exceeding 120 kJ/kg dry matter.

19. Defibrated (fluffed) cellulose pulp for production of an absorbent material intended to be included as a main constituent in absorbent products, **characterised in that** it has been treated in a water suspension at 4<pH<8 with a non polymeric aluminium salt, which is water soluble, during a time period of at least 2 min, whereafter the fibre pulp has been formed to a web which has been dewatered, dried and defibrated, that the defibrated pulp has a moisture content of more than 4.5% and not more than 15%, that the freed fibres have a knot content of at most 8%, and that the defibrated material has a network strength of at least 5 N/1g.

20. Defibrated cellulose pulp according to claim 19, **characterised in that** the freed fibres have a knot content of at most 7%.

21. Defibrated cellulose pulp according to any of claims 19-20, **characterised in that** it has a network strength of at least 6 N/1g.

22. Defibrated cellulose pulp according to any of claims 19-21, **characterised in that** it has a knot content of at most 8% and a network strength of at least 6 N/1g.

23. Absorption product, i.e. a product for absorption of body fluids, comprising an absorption material consisting of defibrated and compressed cellulose pulp according to any of claims 19-22, **characterised in that** it contains less than 60% super-absorbent and has a dry density of between 150-500 kg/m³.

24. Absorption product according to claim 23, **characterised in that** it contains super-absorbent and that it is compressed to a density of at least 200 kglm³.

## Patentansprüche

1. Verfahren zur Herstellung eines Zellstoffs, der geeignet ist, zur Herstellung eines saugfähigen Materials, das dazu vorgesehen ist, als ein Bestandteil in saugfähige Produkte eingebunden zu werden, zerfasert (geflockt) zu werden, **dadurch gekennzeichnet, daß** zumindest ein nichtpolymeres Aluminiumsalz, das wasserlöslich ist, zu einem Bestand an Zellstoffasern in einer Wassersuspension hinzugefügt wird, daß dieser Faserbrei zu einer Faserstoffbahn gebildet wird, die entwässert und getrocknet wird, und daß sein Feuchtigkeitsgehalt spätestens vor der Zerfaserung auf mehr als 8 %, jedoch auf nicht mehr als 20 % eingestellt wird.

2. Verfahren nach Anspruch 1, **dadurch gekennzeichnet, daß** der Feuchtigkeitsgehalt spätestens vor der Zerfaserung auf zwischen 9 und 15 %, vorzugsweise auf mindestens 10 % eingestellt wird.

3. Verfahren zur Herstellung eines zerfaserten Zellstoffs, der geeignet ist, zur Herstellung von saugfähigem Material, das dazu vorgesehen ist, als ein Bestandteil in saugfähige Produkte eingebunden zu werden, benutzt zu werden, **dadurch gekennzeichnet, daß** mindestens ein nichtpolymeres Aluminiumsalz, das wasserlöslich ist, zu einem Bestand an Zellstoffasern in einer Wassersuspension hinzugefügt wird, daß dieser Faserbrei zu einer Faserstoffbahn gebildet wird, die entwässert, getrocknet, zerfasert und verdichtet wird, und daß sein Feuchtigkeitsgehalt bei der Verdichtung, die auf die Zerfaserung folgt, auf mehr als 4,5 %, jedoch auf nicht mehr als 15 % eingestellt wird.

4. Verfahren nach Anspruch 3, **dadurch gekennzeichnet, daß** der Feuchtigkeitsgehalt bei der Verdichtung, die auf die Zerfaserung folgt, so eingestellt wird, daß er mehr als 5 %, jedoch nicht mehr als 12 % beträgt.

5. Verfahren nach einem der Ansprüche 1 bis 4, **dadurch gekennzeichnet, daß** der pH des Bestandes auf 4 < pH < 8, vorzugsweise auf pH 6 ± 1, passend auf einen pH zwischen 5,5 und 6,5 eingestellt wird.

6. Verfahren nach Anspruch 5, **dadurch gekennzeichnet, daß** das Aluminiumsalz mindestens ein nichtpolymeres Aluminiumsalz ist, das wasserlöslich ist und in einer Wasserlösung bei dem pH mit Aluminium mindestens einen Hydroxokomplex von dem Typ Al(OH)ₙ^{x} bildet, wobei n eine Zahl zwischen 1 und 3 ist und x 0, + oder 2+ ist, daß das Salz in der Wasserlösung dazu veranlaßt wird, bei dem pH während eines Zeitraums von mindestens 2 Minuten auf die Zellstoffasern in der Suspension einzuwirken und daß der Faserbrei danach zu einer Faserstoffbahn gebildet wird, die entwässert und getrocknet wird.

7. Verfahren nach Anspruch 6, **dadurch gekennzeichnet, daß** der Hydroxokomplex einer oder mehrere der Komplexe ist, die Al(OH)₃⁰, Al(OH)₂⁺ und Al (OH)²⁺ umfassen.

8. Verfahren nach einem der Ansprüche 5 bis 7, **dadurch gekennzeichnet, daß** das Salz in der Wasserlösung veranlaßt wird, bei dem pH während eines Zeitraums von 5 bis 60 min auf die Zellstoffasern einzuwirken.

9. Verfahren nach einem der Ansprüche 1 bis 8, **dadurch gekennzeichnet, daß** das Salz mindestens eines der Salze Aluminiumsulfat, Aluminiumnitrat, Aluminiumhydroxid, Aluminiumoxidhydroxid, Aluminiumoxidchlorid oder anderer Salze nichtpolymerer Aluminiumverbindungen ist, das in einer Säure oder einer Base gelöst werden kann, um in Verbindung mit der nachfolgenden pH-Einstellung in den Bereich 4 < pH < 8 die aktiven Aluminiumhydroxokomplexe zu bilden.

10. Verfahren nach Anspruch 9, **dadurch gekennzeichnet, daß** das Salz Aluminiumsulfat ist.

11. Verfahren nach einem der vorangehenden Ansprüche, **dadurch gekennzeichnet, daß** das Aluminiumsalz dem Bestand in einer Menge zugegeben wird, die 3 bis 24 g Al/kg Zellstoff (Fasern), vorzugsweise in einer Menge, die 6 bis 12 g Al/kg Zellstoff entspricht.

12. Verfahren nach einem der Ansprüche 1 bis 11, **dadurch gekennzeichnet, daß** der Zellstoff ein chemisch aufgeschlossener Zellstoff ist.

13. Verfahren nach Anspruch 12, **dadurch gekennzeichnet, daß** der Zellstoff ein gebleichter (delignifizierter) Zellstoff ist, der gemäß dem Sulfatzellstoffverfahren hergestellt worden ist.

14. Verfahren nach einem der Ansprüche 12 bis 13, **dadurch gekennzeichnet, daß** der Zellstoff eine Mischung aus chemisch aufgeschlossenem Zellstoff und CTMP ist.

15. Verfahren nach einem der Ansprüche 12 bis 14, **dadurch gekennzeichnet, daß** der Zellstoff mit synthetischen Fasern, einschließlich des Typs synthetischer Fasern, die zu der Gruppe der Rayon-, Polyester-, Polypropylen-, Polyethen- oder der sogenannten Verbundfasern gehören, die durch Wärme aktiviert werden können, abgemischt wird.

16. Zellstoff, der geeignet ist, zur Herstellung eines saugfähigen Materials, das dazu vorgesehen ist, als ein Hauptbestandteil in saugfähige Produkte einbezogen zu werden, zerfasert (geflockt) zu werden, **dadurch gekennzeichnet, daß** er in einer Wassersuspension bei 4 < pH < 8 mit einem nichtpolymeren Aluminiumsalz, das wasserlöslich ist, während eines Zeitraums von mindestens 2 min behandelt worden ist, wonach der Faserbrei zu einer Faserstoffbahn gebildet worden ist, die entwässert, getrocknet und deren Feuchtigkeitsgehalt auf mehr als 8 %, jedoch auf nicht mehr als 20 % Feuchtigkeitsgehalt eingestellt worden ist.

17. Zellstoff nach Anspruch 16, **dadurch gekennzeichnet, daß** er einen Feuchtigkeitsgehalt von mindestens 10 % aufweist.

18. Zellstoff nach Anspruch 16 oder 17, **dadurch gekennzeichnet, daß** die Fasern in dem derart behandelten Zellstoff durch Zerfaserung bei dem Feuchtigkeitsgehalt mit einer zugeführten Zerfaserungsenergie, die 130 kJ/kg Trockenmasse, vorzugsweise 120 kJ/kg Trockenmasse nicht übersteigt, zerfasert werden können.

19. Zerfaserter (gefleckter) Zellstoff zur Herstellung eines saugfähigen Materials, das dazu vorgesehen ist, als ein Hauptbestandteil in saugfähige Produkte einbezogen zu werden, **dadurch gekennzeichnet, daß** er in einer Wassersuspension bei 4 < pH < 8 mit einem nichtpolymeren Aluminiumsalz, das wasserlöslich ist, während eines Zeitraums von mindestens 2 min behandelt worden ist, wonach der Faserbrei zu einer Faserstoffbahn gebildet worden ist, die entwässert, getrocknet und zerfasert worden ist, daß der zerfaserte Zellstoff einen Feuchtigkeitsgehalt von mehr als 4,5 % und nicht mehr als 15 % aufweist, daß die befreiten Fasern einen Knotengehalt von höchstens 8 % aufweisen und daß das zerfaserte Material eine Netzwerkfestigkeit von mindestens 5 N/1g aufweist.

20. Zerfaserter Zellstoff nach Anspruch 19, **dadurch gekennzeichnet, daß** die befreiten Fasern einen Knotengehalt von höchstens 7 % aufweisen.

21. Zerfaserter Zellstoff nach einem der Ansprüche 19 bis 20, **dadurch gekennzeichnet, daß** er eine Netzwerkfestigkeit von mindestens 6 N/1g aufweist.

22. Zerfaserter Zellstoff nach einem der Ansprüche 19 bis 21, **dadurch gekennzeichnet, daß** er einen Knotengehalt von höchstens 8 % und eine Netzwerkfestigkeit von mindestens 6 N/1g aufweist.

23. Saugfähiges Produkt, d.h. ein Produkt zur Aufsaugung von Körperflüssigkeiten, das ein saugfähiges Material umfaßt, das aus zerfasertem und verdichtetem Zellstoff nach einem der Ansprüche 19 bis 22 besteht, **dadurch gekennzeichnet, daß** es weniger als 60 % Superabsorber enthält und eine Trockendichte von 150 bis 500 kg/m³ aufweist.

24. Saugfähiges Produkt nach Anspruch 23, **dadurch gekennzeichnet, daß** es Superabsorber enthält und daß zu einer Dichte von mindestens 200 kg/m³ verdichtet ist.

## Revendications

1. Procédé pour la préparation d'une pâte cellulosique adaptée à être défibrée (pâte fluff) pour production d'un matériau absorbant destiné à être inclus comme constituant dans des produits absorbants, **caractérisé en ce qu'**au moins du sel d'aluminium non polymérique, qui est soluble dans l'eau, est ajouté à une suspension de pâte de fibres de cellulose dans une suspension d'eau, **en ce que** cette pâte de fibres est formée en un voile qui est débarrassé de son eau et séché, et **en ce que** sa teneur en eau est ajustée pour être supérieure à 8 % mais ne pas excéder 20 % au plus tard avant le défibrage.

2. Procédé selon la revendication 1, **caractérisé en ce que** la teneur en eau est ajustée pour être comprise entre 9 % et 15 %, de préférence pour être d'au moins 10 % au plus tard avant le défibrage.

3. Procédé pour la préparation d'une pâte cellulosique défibrée adaptée à être utilisée pour la production d'un matériau absorbant destiné à être inclus comme constituant dans des produits absorbants, **caractérisé en ce qu'**au moins du sel d'aluminium non polymérique, qui est soluble dans l'eau, est ajouté à une suspension de pâte de fibres de cellulose dans une suspension d'eau, **en ce que** cette pâte de fibres est formée en un voile qui est débarrassé de son eau, séché, défibré et comprimé, et **en ce que** sa teneur en eau est ajustée pour être supérieure à 4,5 % mais ne pas excéder 15 % au plus tard au moment de la compression qui suit le défibrage.

4. Procédé selon la revendication 3, **caractérisé en ce que** la teneur en eau est ajustée pour être supérieure à 5 % mais ne pas excéder 12 % au moment de la compression qui suit le défibrage.

5. Procédé selon l'une quelconque des revendications 1 à 4, **caractérisé en ce que** le pH de la suspension de pâte est ajusté pour se situer dans la plage 4<pH<8, de préférence à un pH 6+/-1, de manière appropriée à un pH entre 5,5 et 6,5.

6. Procédé selon la revendication 5, **caractérisé en ce que** ledit sel d'aluminium est au moins du sel d'aluminium non polymérique, qui est soluble dans l'eau et qui, dans une solution d'eau audit pH, forme au moins un complexe hydroxy avec l'aluminium du type Al(OH)ₙ^{x}, où n est un nombre entre 1 et 3 et x est 0, + ou 2+, **en ce que** ledit sel dans la solution d'eau est amené à agir sur les fibres de cellulose dans ladite suspension audit pH pendant une durée d'au moins 2 minutes, et **en ce que** la pâte de fibres est ensuite formée en un voile qui est débarrassé de son eau et séché.

7. Procédé selon la revendication 6, **caractérisé en ce que** ledit complexe hydroxy est un ou plusieurs des complexes qui composent Al(OH)₃⁰, Al(OH)₂⁺ et Al(OH)²⁺.

8. Procédé selon l'une quelconque des revendications 5 à 7, **caractérisé en ce que** ledit sel dans la solution d'eau est amené à agir sur les fibres de cellulose audit pH pendant une durée de 5 à 60 minutes.

9. Procédé selon l'une quelconque des revendications 1 à 8, **caractérisé en ce que** ledit sel est au moins l'un des sels sulfate d'aluminium, nitrate d'aluminium, hydroxyde d'aluminium, oxyhydroxyde d'aluminium, oxychlorure d'aluminium ou d'autres sels de composés d'aluminium non polymériques qui peuvent être dissous dans un acide ou une base afin de former lesdits complexes hydroxy d'aluminium actifs relativement à l'ajustement ultérieur du pH dans la plage 4<pH<8.

10. Procédé selon la revendication 9, **caractérisé en ce que** le sel est du sulfate d'aluminium.

11. Procédé selon l'une quelconque des revendications précédentes, **caractérisé en ce que** ledit sel d'aluminium est ajouté à la suspension de pâte en une quantité correspondant à 3 à 24 g Al/kg de pâte (fibres), de préférence en une quantité correspondant à 6 à 12 g Al/kg de pâte.

12. Procédé selon l'une quelconque des revendications 1 à 11, **caractérisé en ce que** la pâte cellulosique est une pâte cellulosique chimique.

13. Procédé selon la revendication 12, **caractérisé en ce que** la pâte cellulosique est une pâte cellulosique blanchie (délignifiée) qui a été produite suivant le procédé de cellulose au sulfate.

14. Procédé selon l'une quelconque des revendications 12 et 13, **caractérisé en ce que** la pâte cellulosique est un mélange de pâte chimique et de pâte chimico-thermomécanique (CMTP).

15. Procédé selon l'une quelconque des revendications 12 à 14, **caractérisé en ce que** la pâte cellulosique est mélangée avec des fibres synthétiques, dont le type de fibres synthétiques qui appartiennent au groupe rayonne, polyester, polypropylène, polyéthylène ou fibres de liage qui peuvent être activées par la chaleur.

16. Pâte cellulosique adaptée à être défibrée (pâte fluff) pour production d'un matériau absorbant destiné à être inclus comme constituant principal dans des produits absorbants, **caractérisée en ce qu'**elle a été traitée dans une suspension d'eau à un pH dans la plage 4<pH<8 avec un sel d'aluminium non polymérique, qui est soluble dans l'eau, pendant une durée d'au moins 2 minutes, puis la pâte de fibres a été formée en un voile qui a été débarrassé de son eau, séché et a eu une teneur en eau ajustée pour être supérieure à 8 % mais ne pas excéder 20 %.

17. Pâte cellulosique selon la revendication 16, **caractérisée en ce qu'**elle a une teneur en eau d'au moins 10 %.

18. Pâte cellulosique selon la revendication 16 ou 17, **caractérisée en ce que** les fibres dans la pâte cellulosique ainsi traitée peuvent être défibrées par défibrage à ladite teneur en eau avec une introduction d'énergie de défibrage n'excédant pas 130 kJ/kg de matière sèche, de préférence n'excédant pas 120 kJ/kg de matière sèche.

19. Pâte cellulosique défibrée (pâte fluff) pour production d'un matériau absorbant destiné à être inclus comme constituant principal dans des produits absorbants, **caractérisée en ce qu'**elle a été traitée dans une suspension d'eau à un pH dans la plage 4<pH<8 avec un sel d'aluminium non polymérique, qui est soluble dans l'eau, pendant une durée d'au moins 2 minutes, puis la pâte de fibres a été formée en un voile qui a été débarrassé de son eau, séché et défibré, **en ce que** la pâte défibrée a une teneur en eau de plus de 4,5 % et n'excédant pas 15 %, **en ce que** les fibres libérées ont une teneur en noeuds d'au plus 8 %, et **en ce que** le matériau défibré a une résistance en réseau d'au moins 5 N/1g.

20. Pâte cellulosique défibrée selon la revendication 19, **caractérisée en ce que** les fibres libérées ont une teneur en noeuds d'au plus 7 %.

21. Pâte cellulosique défibrée selon l'une quelconque des revendications 19 et 20, **caractérisée en ce qu'**elle a une résistance en réseau d'au moins 6 N/1g.

22. Pâte cellulosique défibrée selon l'une quelconque des revendications 19 à 21, **caractérisée en ce qu'**elle a une teneur en noeuds d'au plus 8 % et une résistance en réseau d'au moins 6 N/1g.

23. Produit absorbant, c'est-à-dire un produit pour absorber les fluides du corps, comprenant un matériau absorbant consistant en pâte cellulosique défibrée et comprimée selon l'une quelconque des revendications 19 à 22, **caractérisé en ce qu'**il renferme moins de 60 % de superabsorbant et a une densité à sec comprise entre 150 et 500 kg/m³.

24. Produit absorbant selon la revendication 23, **caractérisé en ce qu'**il renferme un superabsorbant et **en ce qu'**il est comprimé à une densité d'au moins 200 kg/m³.
